(19) Europäisches Patentamt
European Patent Office
Office européen des brevets

(11) **EP 3 189 776 A1**

(12) **EUROPEAN PATENT APPLICATION**

(43) Date of publication:
**12.07.2017 Bulletin 2017/28**

(51) Int Cl.:
*A61B 5/00* *(2006.01)* *A61B 6/00* *(2006.01)*
*A61B 8/02* *(2006.01)* *A61B 8/08* *(2006.01)*
*A61B 5/024* *(2006.01)*

(21) Application number: **16150527.6**

(22) Date of filing: **08.01.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventor: **The designation of the inventor has not yet been filed**

(74) Representative: **de Haan, Poul Erik et al**
**Philips International B.V.**
**Philips Intellectual Property & Standards**
**High Tech Campus 5**
**5656 AE Eindhoven (NL)**

(54) **AN APPARATUS AND METHOD FOR GENERATING FETAL HEART RATE DATA**

(57) The invention relates to an apparatus (100) and a corresponding method for generating fetal heart rate data. The apparatus (100) comprises a feature extractor (101) configured for extracting at least one feature from acquired ultrasound signal segments (US). The apparatus (100) also comprises a classifier (102) configured for classifying ultrasound data (UD) into a valid signal group and an invalid signal group on the basis of the extracted at least one feature and a plurality of predefined rules, wherein the ultrasound data (UD) are the acquired ultrasound signal segments (US) or autocorrelations of the acquired ultrasound signal segments (US). The apparatus (100) also comprises a processor (103) configured for generating the fetal heart rate data on the basis of the valid signal group.

FIG. 1

EP 3 189 776 A1

**Description**

FIELD OF THE INVENTION

The invention relates to an apparatus and a method for generating fetal heart rate (FHR) data. The invention further relates to a computer program element for performing the method and a computer readable medium for storing the program element.

BACKGROUND OF THE INVENTION

Fetal monitoring is used to determine whether all the vital organs of a fetus are supplied with sufficient oxygenated blood. Although the direct measure of oxygen saturation of a fetus is challenging, the risk symptoms may be identified by fetal heart rhythm analysis. Fetal heart rate (FHR) is a vital parameter and one of the very few useful fetal signals which can be monitored during pregnancy and labor. Intervention based on FHR monitoring is helpful to reduce newborn academia and other problems.

Ultrasound imaging is commonly used for fetal monitoring noninvasively. An ultrasound transducer is placed on the external surface of a pregnant woman's abdomen. The Doppler shift of the received ultrasound signals is related to the periodic movement of a fetal heart. With advanced signal processing techniques, FHR may be derived from the recorded ultrasound signals.

The fetal heart is small so that it can be a challenge to accurately position the ultrasound beam. Moreover, the orientation of the ultrasound beam with respect to the fetal heart is easily changed, due to various interferences, for example, interferences caused by fetal movement, fetal or maternal breathing, and maternal blood vessels etc. Therefore, the shape of an acquired ultrasound signals and the number of episodes being observed during a single cardiac cycle may vary and a signal may even be absent sometimes. Consequently, the recorded ultrasound signals for fetal monitoring usually contain some information caused by various interferences which is disturbing the derivation of the FHR.

The publication Classification of Fetal Heart Rate Series, ICASSP-88, 1988, discloses a method for classifying FHR signals using different classification methods. Features are extracted from FHR time series and a classification is applied on the basis of these features. The method in the publication is not relevant to FHR generation. By using this method, the above problem still exists.

SUMMARY OF THE INVENTION

It is an object of the invention to provide an apparatus and a method for generating fetal heart rate data with improved reliability.

According to a first aspect of the present invention the object is achieved by an apparatus for generating fetal heart rate data comprising a feature extractor configured for extracting at least one feature from acquired ultrasound signal segments; a classifier configured for classifying ultrasound data into a valid signal group and an invalid signal group on the basis of the extracted at least one feature and a plurality of predefined rules, the ultrasound data are the acquired ultrasound signal segments or autocorrelations of the acquired ultrasound signal segments; and a processor configured for generating the fetal heart rate data on the basis of the valid signal group.

The acquired ultrasound signal segments contain not only the useful components which may be used to derive FHR data, but also various interferences. By using the classifier, the valid signal group is identified from the acquired ultrasound signal segments. The valid signal group has better quality than the invalid signal group, which means signals in the valid signal group contain more useful components to derive FHR data from than signals in the invalid signal group. In sequence, the signals in the valid signal group have better quality than the acquired ultrasound signal segments. Therefore, generating FHR data on the basis of the valid signal group allows achieving better reliability.

In an embodiment of the apparatus according to the present invention, the classifier is configured for classifying the ultrasound data of the valid signal group into at least a first signal group and a second signal group, the quality of the first signal group is better than the quality of the second signal group, the processor comprises a signal improvement module configured for improving the quality of the second signal group. This feature further classifies the valid signal group into a plurality of signal groups according to the signal quality, which allows applying different FHR data generation approaches to the plurality of signal groups. The signal improvement module allows improving the quality of the second signal group, which in sequence improves the quality of the FHR data generated from the second signal group. The overall quality of the FHR data is further improved.

In an embodiment of the apparatus according to the present invention, the ultrasound data is the acquired ultrasound signal segments, the signal improvement module is configured for multiplying a window correction curve with autocorrelations of the second signal group to improve the quality of the second signal group. Multiplying the autocorrelations with the window correction curve results in suppressing the false peaks of the autocorrelations while maintaining the significant maximum of the autocorrelations. This feature allows improving the accuracy of significant maximum detection, which in sequence improving the quality of the ultrasound data. Applying the window correction curve has the advantage of easy use and being effective.

In an embodiment of the apparatus according to the present invention, the ultrasound data is autocorrelations of the acquired ultrasound signal segments, and the signal improvement module is configured for multiplying a window correction curve with the second signal group to improve the quality of the second signal group. Multiplying the autocorrelations with the window correction curve results in suppressing the false peaks of the autocorrelations while maintaining the significant maximum of the autocorrelations. This feature allows improving the accuracy of significant maximum detection, which in sequence improving the quality of the ultrasound data. Applying the window correction curve has the advantage of easy use and being effective.

In an embodiment of the apparatus according to the invention, the at least one feature extracted by the feature extractor comprises a similarity feature whereby the apparatus is arranged to derive the similarity feature by calculating a similarity between the autocorrelations of the acquired ultrasound signal segments and a synthetic template, the synthetic template is generated by repeating the first periodical cycle of the autocorrelation of the acquired ultrasound signal segments. For the autocorrelations of the acquired ultrasound signal segments, the periodicity is related to the quality of the acquired ultrasound signal segments. The better quality of the acquired ultrasound signal segments, the better periodicity of the acquired ultrasound signal segments. This calculation of the first similarity feature allows indicating the periodicity of the autocorrelations of the acquired ultrasound signal segments and the quality of the acquired ultrasound signal segments accordingly.

In an embodiment of the apparatus according to the invention, further comprising a second classifier configured for generating the plurality of predefined rules on the basis of a training database. The plurality of predefined rules are generated based on the available training database, which allows determining the plurality of predefined rules offline.

In an embodiment of the apparatus according to the invention, the training database comprises the at least one feature extracted by the feature extractor from the ultrasound signal segments and given quality annotations of the corresponding ultrasound signal segments This feature enables generating the predefined rules which are relevant to the ultrasound data.

In an embodiment of the apparatus according to the invention, the processor configured for generating the fetal heart rate data by deriving this from autocorrelations calculated on the basis of the valid signal group. Applying this autocorrelation approach characterizes signals in time and frequency domains, which has the advantage of retrieving weak signals to derive FHR data from random noise.

According to a second aspect of the invention, a method for generating fetal heart rate data is provided, comprising the steps of extracting at least one feature from acquired ultrasound signal segments; classifying ultrasound data into a valid signal group and an invalid signal group on the basis of the extracted at least one feature and a plurality of predefined rules, the ultrasound data are the acquired ultrasound signal segments or autocorrelations of the acquired ultrasound signal segments; and generating the fetal heart rate data on the basis of the valid signal group.

In an embodiment of the method according to the invention, the step of classifying further comprises classifying the ultrasound data of the valid signal group into at least a first signal group and a second signal group, the quality of the first signal group is better than the quality of the second signal group, and improving the quality of the second signal group.

In an embodiment of the method according to the invention, the step of extracting comprises deriving a similarity feature by calculating a similarity between autocorrelations of the acquired ultrasound signal segments and a synthetic template, the synthetic template is based on repeating the first periodical cycle of the autocorrelation of the acquired ultrasound signal segments.

In an embodiment of the method according to the invention, the step of classifying comprises generating the plurality of predefined rules by applying a second classifier to a training database.

According to a third aspect of the present invention, a computer program element is provided for controlling a processing unit to perform the method according to the invention for generating fetal heart rate data when being executed by the processing unit.

According to a fourth aspect of the present invention, a computer readable medium is provided having stored thereon the program element according to the present invention.

Detailed explanations and other aspects of the invention will be given below.

BRIEF DESCRIPTION OF THE DRAWINGS

Particular aspects of the invention will now be explained with reference to the embodiments described hereinafter and considered in connection with the accompanying drawings, in which identical parts or sub-steps are designated in the same manner:

Fig. 1 schematically depicts an embodiment of the apparatus according to the present invention;

Fig. 2 schematically illustrates an example of the apparatus according to embodiments of the present invention;

Figs. 3A, 3B and 3C illustrate examples of the signals with different quality levels according to embodiments of the present invention;

Figs. 4A, 4B, 4C and 4D illustrate an example of applying a window correction curve according to an embodiment of the present invention;

Fig. 5 illustrates an example of a synthetic template according to an embodiment of the present invention; and

Fig. 6 schematically depicts a flowchart representing an embodiment of the method according to the present invention.

DETAILED DESCRIPTION OF THE INVENTION

The present invention will be described with respect to particular embodiments and with reference to the drawings, but the invention is not limited thereto but only by the claims. The drawings described are only schematic and are non-limiting. In the drawings, the size of some of the elements may be exaggerated and not drawn to scale for illustrative purposes.

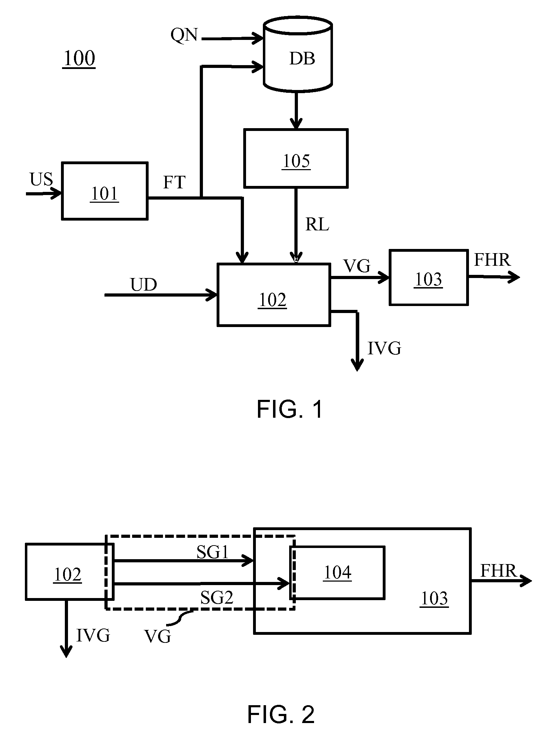

Fig. 1 schematically depicts an apparatus 100 for generating fetal heart rate data. The apparatus 100 comprises a feature extractor 101 configured for extracting at least one feature FT from acquired ultrasound signal segments US. The apparatus 100 furthermore comprises a classifier 102 configured for classifying ultrasound data UD into a valid signal group VG and an invalid signal group IVG on the basis of the extracted at least one feature FT and a plurality of predefined rules RL, the ultrasound data UD are the acquired ultrasound signal segments US or autocorrelations of the acquired ultrasound signal segments US. The apparatus 100 furthermore comprises a processor 103 configured for generating the FHR data on the basis of the valid signal group VG.

The acquired ultrasound signal segments US refer to segments from a sequence of acquired ultrasound signals. The acquired ultrasound signal segments US are acquired by selecting ultrasound signals within a given time window from the sequence of acquired ultrasound signals. For example, by using an adaptive time window with a typical duration of seconds. For fetal heart rate data generation, the acquired ultrasound signal segments US are typically required to comprise 3-4 heart beats. The time duration of the acquired ultrasound signal segment US is dependent on fetal heart beat intervals. A typical range of a fetal heart beat interval is between 0.25 s to 2 s. Therefore, in order to comprise 3-4 heart beats in one segment, the time duration of one segment is between 0.75 s to 8 s.

The at least one feature FT extracted from the acquired ultrasound signal segments US may include time and frequency domain features. The at least one feature FT may be extracted from the acquired ultrasound segments US or may be extracted from autocorrelations of the acquired ultrasound segments US. The at least one feature FT in the time domain may be a well-known feature, for example, signal standard deviation, maximum of autocorrelations, peak-to-peak amplitude of autocorrelations, and half-peak width of autocorrelations. The at least one feature FT in the frequency domain may be a well-known feature, for example, dominant frequency, power of dominant frequency, and half-peak width of power spectral density. The at least one feature FT in the time domain and frequency domains is not limited to the mentioned features.

The classifier 102 may be a plurality of types, for example, decision tree, Bayesian classifier, and k nearest neighbor classifier. These classifiers are known technologies, which build a model through training and assign an input into one group on the basis of the model.

The classifier is formed on the basis of the plurality of predetermined rules RL, which predicts an input signal with specific characteristics to be part of a certain group. The extracted at least one feature FT characterize(s) an acquired ultrasound signal segments. Therefore, taking the extracted at least one feature FT as an input, the classifier 102 delivers a predicted group which the ultrasound data UD belongs to.

The ultrasound data UD is classified into the valid signal group VG and the invalid signal group IVG. The FHR data generation is carried out by the processor 103 only on the basis of the ultrasound data UD classified in the valid signal group VG. The quality of the valid signal group VG is better than the quality of the invalid signal group IVG. The ultrasound data UD classified into the invalid signal group IVG may be labelled as invalid data directly and are not further used for FHR data generation.

The ultrasound data UD classified in the valid signal group VG have good quality, which means having visible periodicity. Instead, the ultrasound data UD classified in the invalid signal group IVG have a poor quality which means non-periodic and usually contaminated with strong motion artifacts.

FHR data generation may be done by some known technologies, for example, autocorrelations, peak detection in time domain and identifying the dominant frequency in the frequency domain.

In an embodiment of the invention, the classifier 102 is configured for classifying the ultrasound data UD of the valid signal group VG into at least a first signal group SG1 and a second signal group SG2. The quality of the first signal group SG1 is better than the quality of the second signal group SG2. The processor 103 comprises a signal improvement module 104 configured for improving the quality of the second signal group SG2.

As illustrated in Fig. 2, the valid signal group VG is further classified into at least the first signal group SG1 and the second signal group SG2. For example, the first signal group SG1 has good quality and the second signal group SG2 has moderate quality. The ultrasound data UD in the first signal group SG1 have clear periodicity; the ultrasound data UD in the second signal group SG2 usually have weak amplitude and/or contain small artifacts.

For the first signal group SG1, FHR may be generated on the basis of the first signal group SG1 directly. The signal improvement module 104 is configured to improve the quality for the second signal group SG2, for example, by applying signal noise reduction or adaptive filtering to the second signal group SG2. After the quality of the second signal group SG2 is improved, the FHR generation is conducted to the second signal improved SG2.

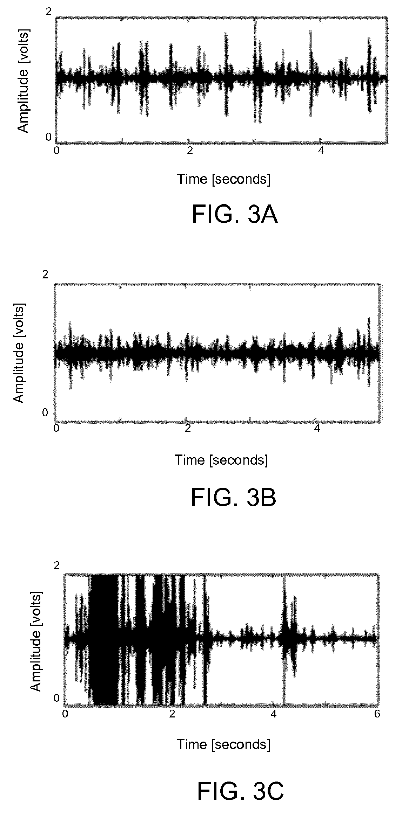

Figs. 3A, 3B and 3C illustrate examples of signals with different quality levels according to embodiments of the present invention. Fig. 3A illustrates an example of an acquired ultrasound signal segment with good quality, which has clear periodicity. Fig. 3B illustrates an example of an acquired ultrasound signal segment with moderate quality, which has a weak amplitude and contains small artifacts. Fig. 3C illustrates an example of an acquired ultrasound signal segment with poor quality, which is non-periodic and contaminated with strong motion artifacts.

In another embodiment of the invention, the ultrasound data UD is the acquired ultrasound signal segment US, and the signal improvement module 104 is configured for multiplying a window correction curve with autocorrelations of the second signal group SG2 to improve the quality of the second signal group SG2.

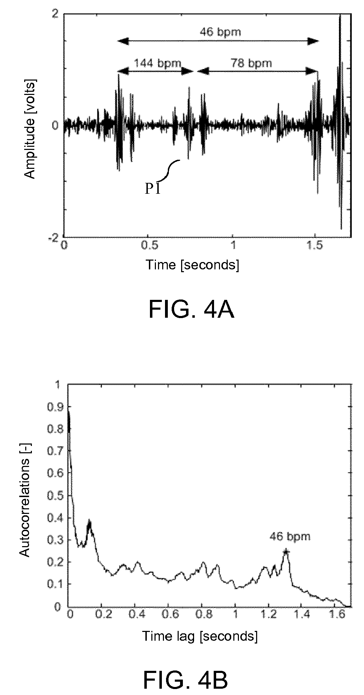

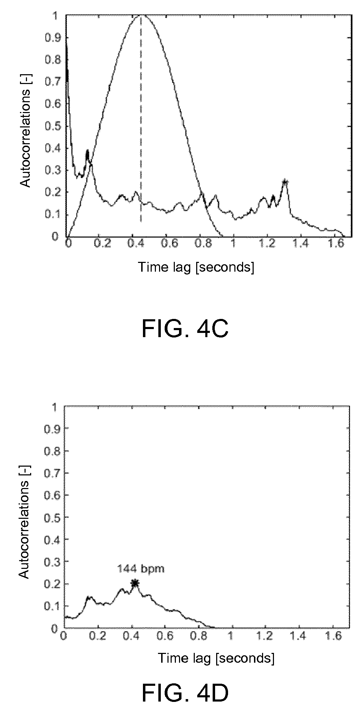

Figs. 4A, 4B, 4C and 4D illustrate an example of applying a window correction curve according to an embodiment of the present invention.

Fig. 4A illustrates an example of an acquired ultrasound signal segment in the second signal group SG2. For the second signal group SG2 with moderate quality, signals have weak amplitude and contain small artifacts. According to Fig. 4A, a first FHR may be detected to be 144 bpm, a second FHR may be detected to be 78 bpm. However, the peak P1 may not be detected due to poor signal quality. As a result, a FHR of 46 bpm is detected, which is a false FHR.

Fig. 4B gives an example of corresponding autocorrelations of the acquired ultrasound signal segment in Fig. 4A. The corresponding autocorrelations have a false peak as 46 bpm. However, the significant maximum of the autocorrelations is 144 bpm.

A window correction curve is illustrated in Fig. 4C, which is centered at the time lag corresponding to the last detected FHR. The autocorrelations of the acquired ultrasound signal segments US in the second signal group SG2 are calculated. The autocorrelations are multiplied with the window correction curve, which results in suppressing the false peaks of the autocorrelations while maintaining the significant maximum in the autocorrelations. Therefore, in the autocorrelations, the port which is close to the last detected FHR is slightly changed, while the other peaks are suppressed.

Fig. 4D illustrates an example of autocorrelations which have been applied by the window correction curve. The autocorrelations in Fig. 4D have a clear peak to be detected, which is a significant maximum as 144 bpm. Therefore, after multiplying the window correction curve, the significant maximum is more accurately and easier to be detected in the autocorrelations. The quality of the autocorrelations of the acquired ultrasound signal segment in the second signal group SG2 is improved.

A common window correction curve is a triangular window curve. Another alternative window correction curve is a hamming window curve. The window correction curve may also be defined by the user.

In another embodiment of the invention, the ultrasound data UD is autocorrelations of the acquired ultrasound signal segments US, and the signal improvement module 104 is configured for multiplying a window correction curve with the second signal group SG2 to improve the quality of the second signal group SG2.

In this embodiment, the autocorrelations of the acquired ultrasound signal segments US is calculated first to derive the ultrasound data UD. As illustrated in Fig. 4B, the corresponding autocorrelations has a false peak as 46 bpm. However, the significant maximum of the autocorrelations is 144 bpm. Then a window correction curve, as illustrated in Fig. 4C, is multiplied with the ultrasound data UD in the second signal group SG2, which is the autocorrelations of the acquired ultrasound signal segments US. It results in suppressing the false peaks of the autocorrelations while maintaining the significant maximum in the autocorrelations. The ultrasound data UD after applied by the window correction curve is illustrated in Fig.4D, which has a clear significant maximum to be detected, i.e., 144 bpm.

Therefore, after multiplying the window correction curve, the significant maximum is more accurately and easier to be detected. The quality of the ultrasound data UD in the second signal group SG2 is improved.

In another embodiment of the invention, the at least one feature FT extracted by the feature extractor 101 comprises a similarity feature whereby the apparatus is arranged to derive the similarity feature by calculating a similarity between autocorrelations of the acquired ultrasound signal segments US and a synthetic template. The synthetic template is generated by repeating the first periodical cycle of the autocorrelations of the acquired ultrasound signal segments US.

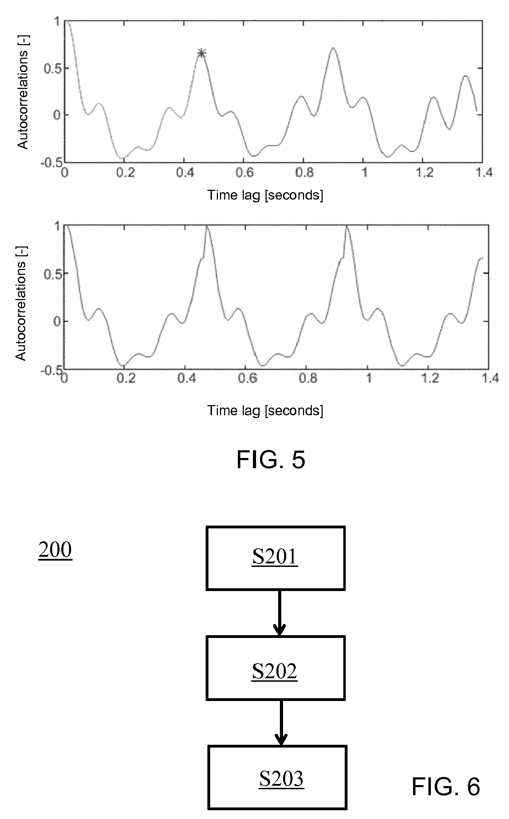

Fig.5 illustrates an example of a synthetic template according to an embodiment of the present invention.

The autocorrelations of the acquired ultrasound signal segments US is a periodic time series. For acquired ultrasound signal segments US with good quality, the autocorrelations of the acquired ultrasound signal segments US have clear periodicity, as illustrated in the upper part of the Fig.5. The first time cycle period of the autocorrelations is extracted and repeated to generate a synthetic template having the same time duration as the autocorrelations, as illustrated in the lower part of the Fig.5. The synthetic template is a synthetic periodic time series of the autocorrelations.

If the autocorrelations have clear periodicity, the similarity between the autocorrelations and the synthetic periodic time series of the autocorrelations is high, which means the similarity between the autocorrelations and the synthetic template is high. Therefore, the similarity feature is defined to calculate the similarity between the autocorrelations and the synthetic template. The similarity between the autocorrelations and the synthetic template may be calculated by know technologies, for example, cross correlation. The similarity feature is defined to indicate whether the autocorrelations have clear periodicity. The larger similarity feature value, the clearer periodicity of the autocorrelations. The clearer periodicity of the autocorrelations, the better quality of the acquired ultrasound signal segments US.

In another embodiment of the invention, the apparatus 100 further comprises a second classifier 105 configured for generating the plurality of predefined rules RL on the basis of a training database DB, as illustrated in Fig.1.

The predefined rules RL may be generated offline, which means not in parallel when acquiring ultrasound signals. The predefined rules RL are generated and then is provided to the classifier 102 for classifying the valid signal group VG and the invalid signal group IVG.

The second classifier 105 may be a plurality of types, for example, decision tree, Bayesian classifier, and k nearest neighbor classifier.

In another embodiment of the invention, the training database DB comprises the at least one feature FT extracted by the feature extractor 101 from the ultrasound signal segments US and given quality annotations QN of the corresponding ultrasound signal segments US, as illustrated in Fig.1.

Each ultrasound signal segment has a corresponding quality annotation. The quality annotations QN may be defined by a user. The quality annotations QN comprises the quality level of good, moderate and poor.

In another embodiment of the invention, the processor 103 configured for generating the fetal heart rate data by deriving from autocorrelation calculated on the basis of the valid signal group VG.

The autocorrelation defines the correlation between a signal and a time-delayed version of the signal itself. The autocorrelation enhances periodic components of a signal while suppressing the random noise of the signal. The autocorrelation of the acquired ultrasound signal segments US in the valid signal group VG is calculated on the basis of the valid signal group VG.

In order to derive FHR data from the autocorrelations, the time lag of the first significant maximum of the autocorrelations is detected, which is the period of the acquired ultrasound signal segment US. Then, the FHR data is calculated according to the below formula:

$$FHR = (60 \times fs) / TL \tag{1}$$

Herein fs denotes the predetermined sampling frequency of the acquired ultrasound signal segments US, TL denotes the period of the acquired ultrasound signal segments US.

Fig. 6 schematically depicts a flowchart of a method 200 for generating fetal heart rate data according to the invention.

The method 200 comprises a step of extracting S201 at least one feature FT from acquired ultrasound signal segments US. The method 200 also comprises a step of classifying S202 ultrasound data UD into a valid signal group VG and an invalid signal group IVG on the basis of the extracted at least one feature FT and a plurality of predefined rules RL, the ultrasound data UD are the acquired ultrasound signal segments US or autocorrelations of the acquired ultrasound signal segments US. The method 200 also comprises a step of generating S203 the fetal heart rate data on the basis of the valid signal group VG.

In an embodiment, the step of classifying S202 further comprises a step of classifying the ultrasound data UD of the valid signal group VG into at least a first signal group SG1 and a second signal group SG2, the quality of the first signal group SG1 is better than the quality of the second signal group SG2. The step of classifying S202 also comprises a step of improving the quality of the second signal group SG2.

In another embodiment, the step of extracting S201 comprises a step of calculating a similarity between autocorrelations of the acquired ultrasound signal segments US and a synthetic template, the synthetic template is based on repeating the first periodical cycle of the autocorrelation of the acquired ultrasound signal segments US.

In another embodiment, the step of classifying S202 comprises a step of generating the plurality of predefined rules RL by applying a second classifier to a training database DB.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. An apparatus (100) for generating fetal heart rate data, comprising:

   - a feature extractor (101) configured for extracting at least one feature (FT) from acquired ultrasound signal segments (US);
   - a classifier (102) configured for classifying ultrasound data (UD) into a valid signal group (VG) and an invalid signal group (IVG) on the basis of the extracted at least one feature (FT) and a plurality of predefined rules (RL), wherein the ultrasound data (UD) are the acquired ultrasound signal segments (US) or autocorrelations of the acquired ultrasound signal segments (US); and
   - a processor (103) configured for generating the fetal heart rate data on the basis of the valid signal group (VG).

2. An apparatus as claimed in claim 1, wherein the classifier (102) is configured for classifying the ultrasound data (UD) of the valid signal group (VG) into at least a first signal group (SG1) and a second signal group (SG2), wherein the quality of the first signal group (SG1) is better than the quality of the second signal group (SG2), wherein the processor (103) comprises a signal improvement module (104) configured for improving the quality of the second signal group (SG2).

3. An apparatus as claimed in claim 2, wherein the ultrasound data (UD) is the acquired ultrasound signal segments (US), and wherein the signal improvement module (104) is configured for multiplying a window correction curve with autocorrelations of the second signal group (SG2) to improve the quality of the second signal group (SG2).

4. An apparatus as claimed in claim 2, wherein the ultrasound data (UD) is autocorrelations of the acquired ultrasound signal segments (US), and wherein the signal improvement module (104) is configured for multiplying a window correction curve with the second signal group (SG2) to improve the quality of the second signal group (SG2).

5. An apparatus as claimed in claim at least one of claims 1 to 4, wherein the at least one feature (FT) extracted by the feature extractor (101) comprises a similarity feature whereby the apparatus is arranged to derive the similarity feature by calculating a similarity between autocorrelations of the acquired ultrasound signal segments (US) and a synthetic template, wherein the synthetic template is generated by repeating the first periodical cycle of the autocorrelations of the acquired ultrasound signal segments (US).

6. An apparatus as claimed in at least one of claims 1 to 4, further comprising a second classifier (105) configured for generating the plurality of predefined rules (RL) on the basis of a training database (DB).

7. An apparatus as claimed in claim 6, wherein the training database (DB) comprises the at least one feature (FT) extracted by the feature extractor (101) from the ultrasound signal segments (US) and given quality annotations (QN) of the corresponding ultrasound signal segments (US).

8. An apparatus as claimed in at least one of claims 1 to 3, wherein the processor (103) is configured for generating the fetal heart rate data by deriving this from autocorrelations calculated on the basis of the valid signal group (VG).

9. A method (200) for generating fetal heart rate data, comprising the steps of:

   - extracting (S201) at least one feature (FT) from acquired ultrasound signal segments (US);
   - classifying (S202) ultrasound data (UD) into a valid signal group (VG) and an invalid signal group (IVG) on the basis of the extracted at least one feature (FT) and a plurality of predefined rules (RL), wherein the ultrasound data (UD) are the acquired ultrasound signal segments (US) or autocorrelations of the acquired ultrasound signal segments (US); and
   - generating (S203) the fetal heart rate data on the basis of the valid signal group (VG).

10. A method as claimed in claim 9, wherein the step of classifying (S202) further comprises:

- classifying the ultrasound data (UD) of the valid signal group (VG) into at least a first signal group (SG1) and a second signal group (SG2), wherein the quality of the first signal group (SG1) is better than the quality of the second signal group (SG2);
- improving the quality of the second signal group (SG2).

11. A method as claimed in claims 9 or 10, wherein the step of extracting (S201) comprises:

- deriving a similarity feature by calculating a similarity between autocorrelations of the acquired ultrasound signal segments (US) and a synthetic template, wherein the synthetic template is based on repeating the first periodical cycle of the autocorrelation of the acquired ultrasound signal segments (US).

12. A method as claimed in claim 9, 10 or 11, wherein the step of classifying (S202) comprises:

- generating the plurality of predefined rules (RL) by applying a second classifier to a training database (DB).

13. A computer program element for controlling a program controlled processing unit to perform the method as claimed in any of the claims 9-12 when being executed by the processing unit.

14. A computer readable medium having stored thereon the program element of claim 13.

FIG. 1

FIG. 2

FIG. 3A

FIG. 3B

FIG. 3C

FIG. 4A

FIG. 4B

FIG. 4C

FIG. 4D

FIG. 5

FIG. 6

Europäisches
Patentamt

European
Patent Office

Office européen
des brevets

# EUROPEAN SEARCH REPORT

Application Number

EP 16 15 0527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/208009 A1 (FU YONGJI [US] ET AL) 25 August 2011 (2011-08-25) | 1,2,5, 9-11,13, 14 | INV. A61B5/00 A61B6/00 A61B8/02 A61B8/08 A61B5/024 |
| Y | * paragraph [0064]; figure 13 * | 5-7,11, 12 | |
| X | WO 2012/061827 A2 (WEST WIRELESS HEALTH INST [US]; ROHAM MASOUD [US]; SALDIVAR ENRIQUE [U) 10 May 2012 (2012-05-10) | 1,5,6,8, 9,11-14 | |
| Y | * paragraphs [0121], [0122]; figure 6 * | 5-7,11, 12 | |
| X | EP 2 767 228 A1 (EDAN INSTRUMENTS INC [CN]) 20 August 2014 (2014-08-20) | 1-6,9-14 | |
| Y | * paragraph [0028] * | 5-7,11, 12 | |
| X | WO 2014/029986 A1 (FERNANDES & CO GLOBAL HEALTHCARE SOLUTIONS LTD [GB]) 27 February 2014 (2014-02-27) | 1,5,6,9, 11-14 | |
| Y | * page 10, line 33-35 * | 5-7,11, 12 | TECHNICAL FIELDS SEARCHED (IPC) |
| X | DE 103 45 717 A1 (TRIUM ANALYSIS ONLINE GMBH [DE]) 28 April 2005 (2005-04-28) | 1,5,6,9, 11-14 | A61B |
| Y | * paragraph [0035] * | 5-7,11, 12 | |
| X | WO 2013/088314 A1 (KONINKL PHILIPS ELECTRONICS NV [NL]) 20 June 2013 (2013-06-20) | 1,5,6,9, 11-14 | |
| Y | * page 8, line 24-32 * | 5-7,11, 12 | |
| Y | EP 2 221 637 A2 (GEN ELECTRIC [US]) 25 August 2010 (2010-08-25) * paragraph [0052] * | 5,11 | |

-/--

The present search report has been drawn up for all claims

| Place of search | Date of completion of the search | Examiner |
|---|---|---|
| Munich | 9 June 2016 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 103 462 642 A (UNIV GUANGDONG TECHNOLOGY) 25 December 2013 (2013-12-25) * paragraph [0005] * ----- | 5,11 | |
| Y | US 2004/133115 A1 (HAMILTON EMILY F [CA] ET AL) 8 July 2004 (2004-07-08) * paragraphs [0193] - [0194] * ----- | 6,7,12 | |
| Y | WO 2010/143113 A1 (KONINKL PHILIPS ELECTRONICS NV [NL]; PHILIPS INTELLECTUAL PROPERTY [DE] 16 December 2010 (2010-12-16) * page 15, line 4-7 * ----- | 6,12 | |

**TECHNICAL FIELDS
SEARCHED (IPC)**

The present search report has been drawn up for all claims

| Place of search | Date of completion of the search | Examiner |
|---|---|---|
| Munich | 9 June 2016 | Anscombe, Marcel |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.                EP 16 15 0527

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2016

| Patent document cited in search report | | Publication date | Patent family member(s) | | Publication date |
|---|---|---|---|---|---|
| US 2011208009 | A1 | 25-08-2011 | CN | 102770063 A | 07-11-2012 |
| | | | EP | 2538835 A1 | 02-01-2013 |
| | | | JP | 2013518607 A | 23-05-2013 |
| | | | US | 2011208009 A1 | 25-08-2011 |
| | | | US | 2014025311 A1 | 23-01-2014 |
| | | | WO | 2011105462 A1 | 01-09-2011 |
| WO 2012061827 | A2 | 10-05-2012 | AU | 2011323102 A1 | 23-05-2013 |
| | | | CA | 2816894 A1 | 10-05-2012 |
| | | | EP | 2635191 A2 | 11-09-2013 |
| | | | JP | 2014500742 A | 16-01-2014 |
| | | | KR | 20140035313 A | 21-03-2014 |
| | | | US | 2012232398 A1 | 13-09-2012 |
| | | | WO | 2012061827 A2 | 10-05-2012 |
| EP 2767228 | A1 | 20-08-2014 | CN | 102319064 A | 18-01-2012 |
| | | | EP | 2767228 A1 | 20-08-2014 |
| | | | US | 2014243674 A1 | 28-08-2014 |
| | | | WO | 2013053290 A1 | 18-04-2013 |
| WO 2014029986 | A1 | 27-02-2014 | NONE | | |
| DE 10345717 | A1 | 28-04-2005 | NONE | | |
| WO 2013088314 | A1 | 20-06-2013 | CN | 104114101 A | 22-10-2014 |
| | | | EP | 2790588 A1 | 22-10-2014 |
| | | | JP | 2015500120 A | 05-01-2015 |
| | | | RU | 2014129043 A | 10-02-2016 |
| | | | US | 2014358000 A1 | 04-12-2014 |
| | | | WO | 2013088314 A1 | 20-06-2013 |
| EP 2221637 | A2 | 25-08-2010 | EP | 2221637 A2 | 25-08-2010 |
| | | | US | 2010191118 A1 | 29-07-2010 |
| CN 103462642 | A | 25-12-2013 | NONE | | |
| US 2004133115 | A1 | 08-07-2004 | CA | 2447861 A1 | 01-05-2004 |
| | | | US | 2004133115 A1 | 08-07-2004 |
| WO 2010143113 | A1 | 16-12-2010 | CN | 102458259 A | 16-05-2012 |
| | | | EP | 2440139 A1 | 18-04-2012 |
| | | | JP | 5771200 B2 | 26-08-2015 |
| | | | JP | 2012529329 A | 22-11-2012 |
| | | | RU | 2011153951 A | 20-07-2013 |
| | | | US | 2012083699 A1 | 05-04-2012 |
| | | | WO | 2010143113 A1 | 16-12-2010 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 16 15 0527

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2016

| Patent document cited in search report | Publication date | Patent family member(s) | Publication date |
|---|---|---|---|
| -------------------------------------------------------------------------------------- | | | |

EPO FORM P0459

For more details about this annex : see Official Journal of the European Patent Office, No. 12/82

page 2 of 2